# EUROPEAN PATENT APPLICATION

(11) **EP 3 895 566 A1**
(43) Date of publication of application: **20.10.2021**
(21) Application number: 21167849.5
(22) Date of filing: 12.04.2021
(51) Int. Cl.: A41D 13/11, A62B 7/10, A62B 18/02, A61L 9/20, A62B 23/02

(54) **PROTECTIVE MASK**

(30) Priority: 17.04.2020 IT 202000008227
(71) Applicant: Lit Led Innovation Technology Sagl, 6830 Chiasso (CH)
(72) Inventor: ALIVERTI, Stefano, 22069 Rovellasca, COMO (IT); LIBRANDI, Gianfranco, 22078 Turate, COMO (IT); MOLINARO, Lorenzo, 22078 Turate, COMO (IT)
(74) Representative: Chimini, Francesco

(57) **Abstract**

The object of the present invention consists of a protective mask comprising an antibacterial and antivirus filtering element (12) and incorporating at least one UV-C LED source (18) suitable for applying a suitable amount of UV-C radiation to the filtering element in order to break down the genetic material of bacterial cells and virus particles accumulating in the filtering element.

## Description

The present invention relates to an individual protective mask provided with an antibacterial and antivirus filter and provided with suitable expedients for periodically sterilizing the filter while it is being worn by an operator.

As is known, healthcare workers are exposed to the risk of contracting diseases and infections due to the exposure to viruses and bacteria transmitted by visiting and treated patients.

To avoid the risk of contracting diseases, suitable personal protective devices are required to prevent viruses and bacteria from coming into contact with the operator. One of the devices used consists of protective masks provided with filtering structures capable of passing the air necessary for breathing and at the same time blocking the passage of particles, viruses and bacteria.

In a typical configuration, the filtering element consists of a material having a structure suitable for blocking the passage of particles having a diameter greater than a predefined limit. The particles are not destroyed, but are retained and accumulate inside the filter.

The filtering is never complete, and in fact it is expressed as a percentage of blocked particles having a size which is greater than a certain diameter. For example, according to current regulations a standard mask classified FFP3 is capable of blocking over 99% of particles having a diameter greater than 0.6um.

During use, with the accumulation of viruses and bacteria inside the filter, the viral load which accumulates in the filter gradually increases the risk of transfer to the inner side and therefore to the operator.

Most masks can no longer be reused once they have been removed from the face. In fact, they are therefore disposable protective devices.

In order to try to overcome such a drawback, masks provided with a replaceable filtering element have been specially developed. Such masks have a window which can be opened to replace the filter inserted therein.

However, the maneuver of replacing the filter requires time and above all precautions, to avoid the risk of contamination from viruses and/or bacteria accumulated on the outer side.

In many situations it may be necessary to perform this maneuver even several times during the working day.

The object of the present invention is to propose a protective mask capable of at least partially obviating the drawbacks mentioned above.

Such an object is achieved by a protective mask in accordance with claim 1. The dependent claims describe preferred or advantageous embodiments of the mask.

In accordance with claim 1, the protective mask incorporates a LED source capable of emitting UV-C radiation suitable for sterilizing the filtering structure of the mask.

Therefore, the protective mask comprises an antibacterial and antivirus filtering element, and incorporates at least one UV-C LED source suitable for applying a suitable amount of UV-C radiation to the filtering element in order to break down the genetic material of bacterial cells and virus particles accumulating in the filtering element.

In an embodiment, the UV-C LED source is positioned so as to irradiate at least one outer surface of the filtering element.

In an embodiment, the UV-C LED source is positioned so as to irradiate the side of the filtering element which faces the operator wearing the mask.

In an embodiment, the mask integrates two UV-C LED sources, one adapted to irradiate a particular side of two opposite sides of the filtering element.

In an embodiment, the material of the filtering element is selected so as to allow at least some of the UV-C radiation to pass through such a material.

In an embodiment, the UV-C LED source is powered by a battery.

In an embodiment, the mask comprises a first shielding element suitable to protect the operator wearing the mask from the UV-C radiation.

In an embodiment, the mask comprises a second shielding element suitable to reduce the emission of the UV-C radiation towards the outside of the mask.

In an embodiment, the UV-C LED source is actuatable manually.

In an embodiment, the UV-C LED source is actuatable automatically by means of a timer which, during use, applies sterilizing pulses of UV-C radiation at predefined time intervals.

Therefore, the mask according to the invention, by neutralizing the bacterial load accumulated inside the filter, reduces the risk of exposure to viruses/bacteria of the operator wearing it, with the double advantage of reducing the periodic replacements of the filter in the span of the working day and at the same time reducing the risk of contamination by the staff using the mask.

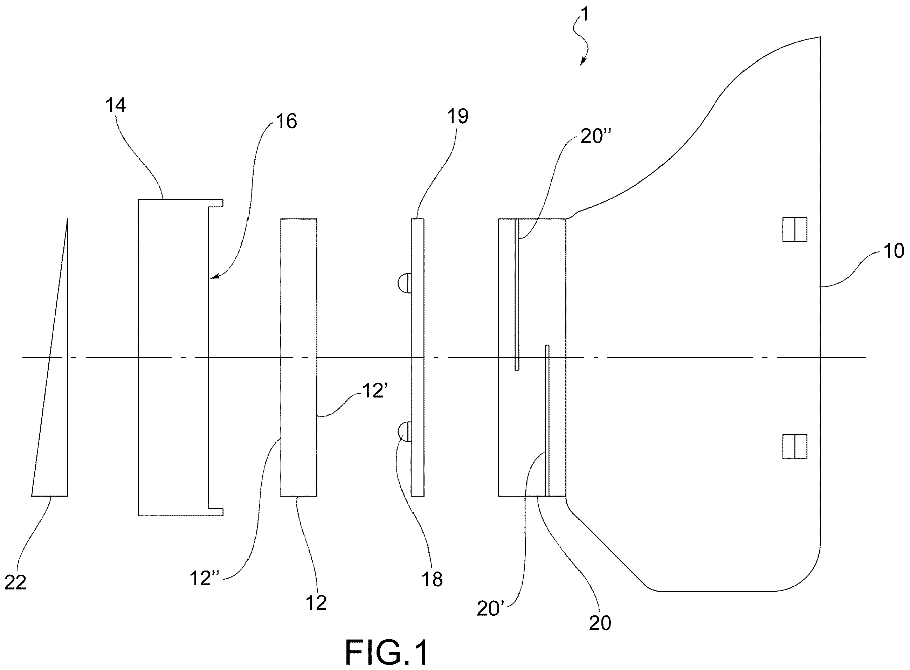

The features and advantages of the protective mask according to the invention will be apparent from the following description which illustrates preferred embodiments thereof, given by way of indicative, nonlimiting examples, with reference to the accompanying figure 1, which shows a view of the mask with separate parts in a possible embodiment.

Numeral 1 in said drawings indicates a protective mask according to the invention, as a whole.

The mask 1 comprises a mask body 10 wearable by an operator, a filtering element 12 suitable for blocking the particles of a bacterium or a virus, and a mask stopper 14 which is connectable, preferably removably, to the mask body 10, so as to form a filter seat 16 together with the mask body 10 suitable to house the filtering element 12.

In accordance with the invention, the mask 10 further incorporates at least one UV-C LED source 18 suitable for applying a suitable amount of UV-C radiation to the filtering element 12 in order to break down the genetic material of bacterial cells and virus particles accumulating in the filtering element 12.

UV-C radiation is intended as optical radiations having a wavelength in the range 100nm-280nm, therefore having sufficient energy to break down the DNA of the bacteria and viruses, with the result of neutralizing them and thus making them harmless.

In an embodiment, the UV-C LED source 18 is housed in the filter seat 16.

In an embodiment shown in figure 1, the UV-C source 18 is positioned and oriented so as to irradiate the inner side 12' of the filtering element 12, i.e., the one facing the operator wearing the mask, destroying the bacteria and/or viruses which are more likely to be breathed.

In a variant embodiment, two UV-C sources 18 can be used, one facing a respective side 12', 12" of the two opposite sides of the filtering element 12.

In a variant embodiment, the UV-C LED source could also be positioned and turned so as to irradiate the outer side 12" of the filtering element, blocking the load retained on the outer surface.

For example, the location and/or number of UV-C LED sources can depend on the type of filtering material used.

The material of the filtering element could also be advantageously selected so as to allow at least some of the UV-C radiation to pass through such a material. Thereby it would be possible to completely sterilize the filter with a single UV-C source applied on a single side.

In an embodiment, the UV-C LED source 18 is mounted on an electronic board 19 which also forms the electronic control circuit of the UV-C LED source 18.

The UV-C LED source 18 can be easily battery powered, making the system integrable in the mask structure. The battery can be housed in a special battery compartment or supported by the electronic board 19.

In an embodiment, the mask 1 comprises a first shielding element 20 suitable to protect the operator wearing the mask from the UV-C radiation. Such a first shielding element 20 can for example be connected to the mask body 10, as shown in figure 1, so as to be interposed between the mask body and the UV-C LED source.

In an embodiment illustrated in figure 1, the shielding element 20 includes at least two diaphragms 20', 20" which allow the passage of air and at the same time block the passage of the UV-C radiation. In fact, such diaphragms are partially overlapping each other in the direction of the optical path of the UV-C radiation, but spaced apart. Thereby, the mutual positioning of the diaphragms allows to block the UV-C radiation which follows an optical path, while allowing the passage of air which can go around the diaphragms and therefore pass in the space formed by the spacing between the diaphragms.

In some embodiments, porous materials or fabrics permeable to air but opaque to UV-C radiation can also be used.

Furthermore, the mask 1 can be provided with a second shielding or protective element 22, suitable to reduce the emission of the UV-C radiation towards the outside of the mask. For example, such a second shielding or protective element 22 can be applied to the mask stopper 14, as shown in figure 1.

The UV-C LED source can be actuated manually or automatically by means of a timer which, during use, applies sterilizing pulses of UV-C radiation at predefined time intervals.

In a practical embodiment, the UV-C LED source 18 is controlled by a PIC16F675 micro controller which actuates the UV-C LED source for 1 second every 10 minutes.

In a practical embodiment, the UV-C LED source 18 consists of four KL265-50T-SM-WD LEDs; given the reduced power supply time, no heat sinks have been included.

In an embodiment, the electronic control circuit comprises a Bluetooth connection module which can be integrated with an application suitable for managing the battery charge, for example also by intervening on the clock to increase and/or decrease the frequency in which the UV-C LED source switches on.

In an embodiment, a signaling LED can be placed on the outside of the mask in an easily visible position to indicate when the UV-C LED source is about to switch on.

The mask 1 has an ergonomic mechanism which is easy to adapt to all faces and can be anchored to the person's face with comfortable elastics.

If included, the battery compartment will be easy to access, so as to give the possibility to quickly and easily change the battery.

A person skilled in the art may make changes and adaptations to the embodiments of the mask according to the invention or can replace elements with others which are functionally equivalent to satisfy contingent needs without departing from the scope of protection of the appended claims. All the features described above as belonging to one possible embodiment may be implemented independently from the other described embodiments.

## Claims

1. A protective mask comprising an antibacterial and antivirus filtering element (12), **characterized in that** said mask incorporates at least one UV-C LED source (18) suitable for applying a suitable amount of UV-C radiation to the filtering element in order to break down the genetic material of bacterial cells and virus particles accumulating in the filtering element.

2. The mask according to claim 1, wherein the UV-C LED source (18) is positioned so as to irradiate an outer surface of the filtering element (12).

3. The mask according to claim 1 or claim 2, wherein the UV-C LED source (18) is positioned so as to irradiate the inner side (12') of the filtering element, which faces the operator wearing the mask.

4. The mask according to any one of the preceding claims, comprising two UV-C LED sources, one suitable to irradiate a particular side of two opposite sides (12', 12") of the filtering element.

5. The mask according to any one of the preceding claims, wherein the material of the filtering element is selected so as to allow at least some of the UV-C radiation to pass through said material.

6. The mask according to any one of the preceding claims, wherein the UV-C LED source is supplied with power by a battery.

7. The mask according to any one of the preceding claims, comprising a first shielding element (20) suitable to protect the operator wearing the mask from the UV-C radiation.

8. The mask according to claim 7, wherein the first shielding element (20) comprises at least two diaphragms (20', 20") that do not allow the UV-C radiation to pass through, said diaphragms being at a spacing so as to allow air to pass therebetween but partially overlapping in the direction of the optical path of the UV-C radiation.

9. The mask according to any one of the preceding claims, comprising a second shielding or protective element (22) suitable to reduce the emission of the UV-C radiation towards the outside of the mask.

10. The mask according to any one of the preceding claims, wherein the UV-C LED source is actuatable manually.

11. The mask according to any one of the preceding claims, wherein the UV-C LED source is actuatable automatically by means of a timer that, during use, applies sterilizing pulses of UV-C radiation at predefined time intervals.

12. The mask according to any one of the preceding claims, comprising a mask body (10) wearable by an operator, and a mask stopper (14) that is connectable or connected to the mask body (10) so as to form a filter seat (16) together with the mask body (10), which seat houses the filtering element (12) and at least one UV-C LED source (18).
